# EUROPÄISCHE PATENTSCHRIFT

(11) **EP 0 923 946 B1**
(45) Veröffentlichungstag und Bekanntmachung des Hinweises auf die Patenterteilung: **15.03.2006**
(21) Anmeldenummer: 98121357.2
(22) Anmeldetag: 10.11.1998
(51) Int. Cl.: A61L 2/06, C12M 1/04, C12M 1/00, B01J 3/04

(54) **Verfahren zum Erzeugen einer desinfizierenden heissen Atmosphäre sowie Vorrichtung**
Method for generating a disinfecting hot atmosphere and apparatus
Méthode et dispositif de génération d' une atmosphère désinfectante chaude

(30) Priorität: 16.12.1997 DE 19755688
(43) Veröffentlichungstag der Anmeldung: 23.06.1999
(73) Patentinhaber: Kendro Laboratory Products GmbH, 63505 Langenselbold (DE)
(72) Erfinder: Paul, Elke, Dr., 64839 Münster (DE); Pieczarek Waldemar, 63486 Bruchköbel (DE); Heeg, Hubert, 63776 Mömbris (DE)
(74) Vertreter: Lang, Friedrich

(56) Entgegenhaltungen:
- DE-C- 4 441 250
- US-A- 1 695 008
- US-A- 4 336 329
- US-A- 5 196 165
- US-A- 5 519 188

## Beschreibung

Die Erfindung betrifft ein Verfahren zum Erzeugen einer desinfizierenden, heißen Atmosphäre gemäß dem Oberbegriff des unabhängigen Anspruchs 1 sowie eine Vorrichtung.

Aus der DE 44 06 632 C1 ist ein Feststoff-Bioreaktor zur Kultur von Mikroorganismen auf festen, partikulären Substraten bekannt, wobei die Temperatur zwischen -27 und +100°C, die relative Feuchte mit Hilfe von mit Ultraschall erzeugtem, kaltem Wasserdampf von 40-99% regelbar ist; dabei wird eine gerichtete in der Zusammensetzung dosierbare (Sauerstoffgehalt 0 bis 100% im Gemisch mit Stickstoff, Kohlendioxid und gegebenenfalls weiteren Gasen einstellbar) und im Volumenstrom regelbare Zwangsströmung der Gasatmosphäre erzeugt, wobei Einrichtungen zur Heißdampfsterilisation des Bioreaktors sowie solche zur Ableitung und Sterilisation von Kondensat und Abluft vorhanden sind.

Als problematisch erweist es sich, daß zur eigentlichen Heißdampf-Sterilisation eine zusätzliche drucklose Heißdämpfversorgung bzw. ein zusätzlicher Anschluß an eine solche Versorgung erforderlich ist.

Weiterhin ist aus der DE 29 24 446 C2 ein Verfahren zur Kultivation von Zellen und Geweben von Menschen und Tieren oder von Mikroorganismen mittels Behältem bekannt, die in einen Brutschrank einzubringen sind, der mit Kohlendioxid, Luft oder Sauerstoff bzw. Stickstoff kontrolliert zu begasen ist, dessen Atmosphäre zu befeuchten ist und auf einer vorgegebenen Temperatur zu halten ist; die Gase werden dabei einzeln oder gemeinsam über einen Sterilfilter der Leitung im Doppelmantel des Brutschrankes einem Bypasskanal zugeführt und dort mit Wasserdampf aus der Leitung vermischt, wobei dieser durch Überhitzen im Verdampfer außerhalb des Nutzraums sterilisiert und abgekühlt wird. Durch eine Regeleinrichtung für die relative Feuchte wird diese im Bereich von etwa 60 bis 95 Prozent gehalten. Dies bedeutet, daß die relative Feuchte nicht nur nahe der Sättigungsgrenze eingestellt werden kann, sondern im weiten Bereich von 60 bis 95 Prozent regelbar ist; der Nutzraum und/oder Bypass einschließlich der Meßfühler ist von einer dem Nutzraum zugeordneten Heizung durch Ausheizen bis zu 180°C sterilisierbar.

Als problematisch erweist sich die verhältnismäßig hohe Desinfektionstemperatur insbesondere im Hinblick auf temperaturempfindliche Bauteile wie beispielsweise Sensoren, Dichtungen oder Umwälzeinrichtungen.

Die Patentschrift US 1,695,008 beschreibt eine Sterilisations- und Inkubationsvorrichtung für bakteriologische Tests an Milch und ähnlichen Proben. Zur Dampfsterilisation wird eine kleine Wassermenge in einem Innenraum der Vorrichtung bereitgestellt und nach Abschluss des Innenraums gegenüber der Umgebungsatmosphäre der Wassermenge Wärme zur Bildung von Wasserdampf zugeführt.

Dokument US 5,196,165 offenbart eine Vorrichtung und ein Verfahren zur Entfernung von Luft aus einem Autoklaven. Für die Sterilisation im Innenraum des Autoklaven wird eine Temperatur zwischen 121 °C und 132 °C als erforderlich angesehen.

Aus der US 5,519,188 geht ein Inkubator mit einem beheizbaren und befeuchtbaren Nutzraum hervor, wobei das den Nutzraum umgebende innere Gehäuse eine Boden-, zwei Seiten-, eine Rück- und eine Deckenwand aufweist. Mittels Heizelementen können die genannten Wände und damit der Nutzraum geheizt werden. Zur Befeuchtung der Nutzraum-Atmosphäre ist eine mit Wasser gefüllte Wanne vorgesehen, die auf der Bodenwand des Nutzraumes angeordnet und mit den der Bodenwand zugeordneten Heizelementen beheizt wird. Dazu können die Heizelemente der Bodenwand von den übrigen Heizelementen unabhängig gesteuert werden.

Aus der DE 4441250 C1 geht ein Brutschrank mit einem beheizbaren und befeuchtbaren Nutzraum hervor. Zur Befeuchtung des Nutzraumes ist Wasser ist einer Wanne vorgesehen, die auf dem Boden des Nutzraumes angeordnet und durch dem Boden zugeordnete Heizkörper beheitzt wird. Weiterhin ist zur Kondensation von Wasserdampf eine zweite, ebenfalls am Boden angeordnete Kondensatwanne vorgesehen.

Aufgabe der Erfindung ist es, ein Verfahren bzw. eine Vorrichtung zur wirksamen Sterilisation - insbesondere im entleerten Innenraum eines Begasungsbrutschrankes - anzugeben, wobei jedoch die bisher üblichen hohen Temperaturen durch Anwendung einer hochfeuchten Desinfektion vermieden werden sollen.

Die Aufgabe wird verfahrensgemäß durch die Merkmale des unabhängigen Anspruchs 1 gelöst.

Aufgrund der hochfeuchten Innenraum-Atmosphäre gemäß der Erfindung erfolgt eine gegenüber trockener Atmosphäre höhere Wärmesensibilisierung der vorhandenen Keime, so daß sich diese in der neunstündigen Desinfektionsphase vorteilhafterweise praktisch alle thermisch abtöten lassen.

In einer bevorzugen Ausgestaltung der Erfindung wird in einem Innenraum mit einem Volumen im Bereich von 15 bis 400 l - vorzugsweise ca. 150 l - eine Wassermenge von wenigstens 300 ml in den Innenraum eingebracht; während der Aufheizphase wird dem Innenraum zwecks Erreichen der Desinfektionstemperatur Wärme über die gesamten Innenraum-Flächen zugeführt; als vorteilhaft erweist sich der automatische Ablauf des Desinfektionsvorganges, wobei eine Dekontamination inklusive aller Einbauten und Sensoren innerhalb des Innenraums erzielt wird; weiterhin erweist es sich als vorteilhaft, daß das Desinfektionsverfahren in einer Atmosphäre vorgenommen wird, deren Druck gegenüber dem der umgebenden Atmosphäre eine vernachlässigbare Differenz aufweist.

Die Aufgabe wird vorrichtungsgemäß durch die Merkmale des unabhängigen Anspruchs 10 gelöst.

Als besonders vorteilhaft erweist es sich, daß aufgrund der verhältnismäßig niedrigen Temperatur die Materialbelastung des Nutzraumes wesentlich geringer ist als bei bekannten Verfahren; weiterhin erweist es sich als vorteilhaft, daß innerhalb des Nutzraumes keine wesenttiche Druckerhöhung gegenüber der Umgebungsatmosphäre erforderlich ist, so daß eventuelle Dichtungsprobleme durch unterschiedlichen Innen- und Außendruck praktisch nicht auftreten.

In einer bevorzugten Ausführungsform bestehen die dem Bodenbereich zugeordneten Temperierelemente aus Heiz- und/oder Kühlelementen; als vorteilhaft erweist es sich, daß sowohl eine rasche Aufheizphase als auch eine verhältnismäßig kurze Kondensationsphase erzielt werden kann.

Weiterhin ist in einer bevorzugten Ausgestaltung der Erfindung der Nutzraum mit einer frontseitigen Öffnung versehen, die mittels einer Innen-Tür gasdicht abschließbar ist, die zur Kondensatvermeidung temperierbar ist; die Innen-Tür kann wenigstens zum Teil transparent ausgebildet sein. Dabei erweist es sich als vorteilhaft, daß eine eventuelle Kondensatbildung an der Innen-Tür und an den Wandungen des Innenraums zur Erreichung trockener Wand-, Decken und Innen-Türflächen weitestgehend vermieden wird.

Der Nutzraum ist der eigentliche Aufnahmeraum für das zu behandelnde Gut, während der Innenraum zusätzlich auch dessen Peripherie wie z.B. einen Ventilator umfaßt.

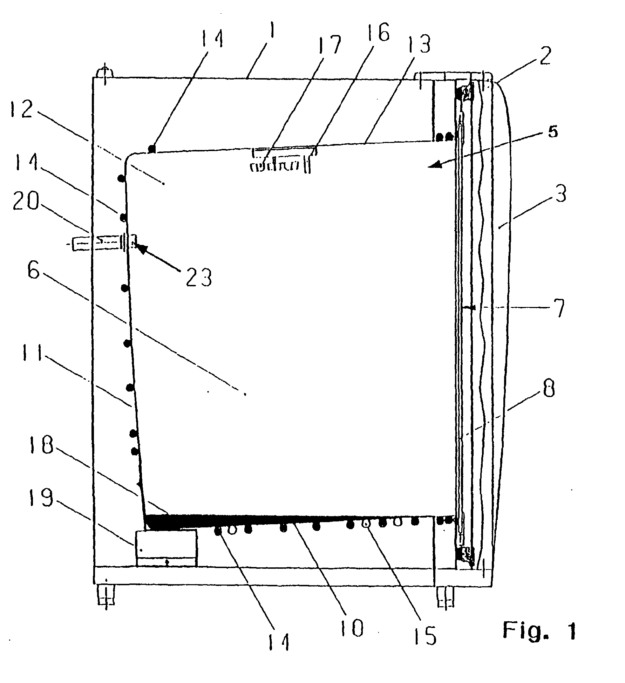

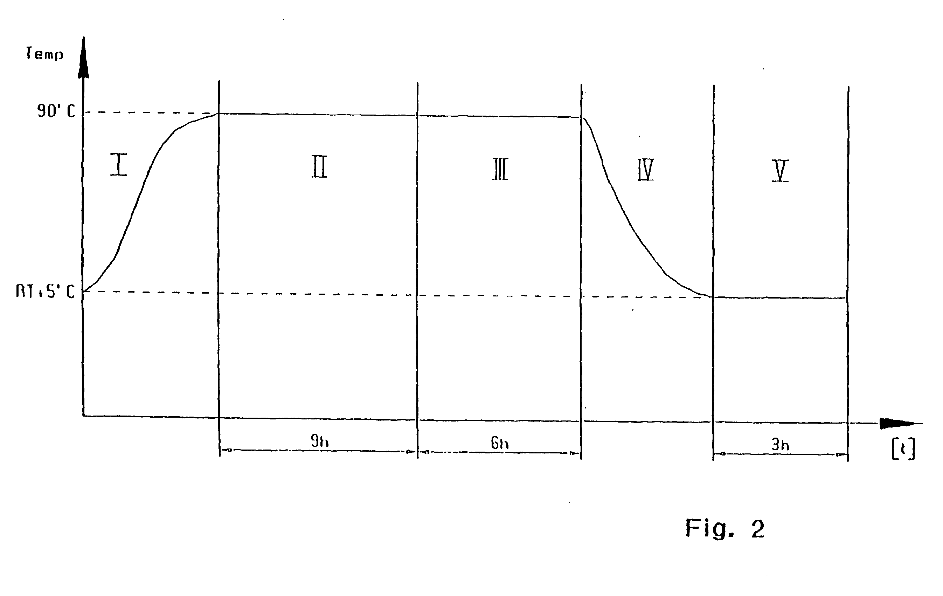

Im folgenden ist der Gegenstand der Erfindung anhand der Figuren 1 und 2 näher erläutert.

Figur 1 zeigt schematisch im Ausschnitt einen Längsschnitt durch den Nutzraum;

Figur 2 zeigt im zeitlichen Diagramm die unterschiedlichen Phasen zur Desinfektion bzw. Inbetriebnahme des Nutzraumes.

Gemäß Figur 1 weist der Begasungsbrutschrank ein Gehäuse 1 auf, dessen Frontseite 2 mit einer Öffnung versehen ist, die mittels Außentür 3 verschlossen wird. Im Innenraum 5 des Gehäuses 1 befindet sich Nutzraum 6, der an seiner zur Frontseite 2 gerichteten Fläche mit einer Öffnung 7 versehen ist, die mittels einer transparenten Innen-Tür 8 mit Dichtfunktion verschließbar ist. Bodenbereich 10, Rückwand 11 und die sich gegenüberliegenden Seitenwände 12 (nur die in der Draufsicht erscheinende Seitenwand ist dargestellt) sowie der Deckenbereich 13 des Nutzraumes sind mit Heizelementen 14 versehen, die sich jeweils außerhalb des Nutzraumes 6 jedoch in engem thermischen Kontakt mit der jeweils angrenzenden Wand bzw. dem Boden und Deckenbereich befinden; die Heizelemente 14 sind über Boden, Decken, Tür- und Wandbereich verteilt, wobei im Bereich des Bodens zusätzlich Kühlelemente 15 angeordnet sein können. Im Inneren des Nutzraumes 6 sind im Deckenbereich 13 ein Temperatursensor 16 sowie ein Ventilator 17 angeordnet.

Im Bodenbereich 10 des Innenraums ist in einer wannenartigen Ausgestaltung 18 des Bodens ein Wasservorrat eingefüllt, welcher für die erforderliche Feuchtigkeit im Desinfektionsverfahren sorgt.

Im Bereich der Rückwand 11 ist eine Öffnung 23 vorgesehen, die mit Hilfe eines Filterstopfens abschließbar ist, wobei der Filterstopfen 20 für einen Druckausgleich zwischen dem Inneren des Nutzraumes 6 und der umgebenen Atmosphäre sorgt, so daß praktisch keine Druckdifferenz auftritt.

Vor dem Desinfektionsvorgang, dessen zeitlicher Verlauf anhand Figur 2 näher erläutert ist, wird der Begasungsbrutschrank abgeschaltet, eventuell vorhandenes Kulturgut aus dem Nutzraum entnommen, sowie der Wasservorrat entleert; weiterhin können manuelle Reinigungsarbeiten vorgenommen werden.

Das eigentliche Desinfektionsverfahren ist anhand der fünf Phasen von I bis V im Temperatur-Zeit-Diagramm gemäß Figur 2 näher erläutert; die Zeit-Achse "t" ist in Phasen I, II, III, IV, V bzw. grob in Stunden (h) unterteilt, die Temperatur-Achse "Temp."in Grad Celsius.

Nach Auffüllen des Bodenbereiches 10 gemäß Figur 1 mit ca. 300 ml Wasser bei einem Innenraumvolumen von ca. 150 I beginnt in Phase I die Aufheizphase mit einem Temperaturanstieg von beispielsweise Raumtemperatur "RT" +5°C (z.B. 37°) auf 90°C (Desinfektionstemperatur). Während dieser Aufheizphase wird durch Wärmezufuhr mittels Heizelemente 14 in Nutzraum 6 die Nutzraumatmosphäre aufgeheizt, wobei zusätzlich durch Heizen der Wassermenge im Bodenbereich von Nutzraum 6 Wasserdampf auftritt, so daß nach Erreichen der Sollwerttemperatur von 90° eine relative Feuchte von mehr als 80% vorherrscht. Die Dauer von Aufheizphase I ist abhängig von der Umgebungstemperatur, des aufzuheizenden Geräts und der Heizleistung.

Nach Erreichen der Sollwerttemperatur von 90°C beginnt der eigentliche Desinfektionsvorgang, welcher hier mit Phase II (9 h) bezeichnet ist, wobei es aufgrund der hochfeuchten Innenraumatmosphäre zu einer hohen Wärmesensibilisierung der vorhandenen Keime kommt und somit in der neunstündigen Desinfektionsphase eine hohe Wärmeleitfähigkeit zu eventuellen Keimen bzw. Bakterienbeständen entsteht; aufgrund der neunstündigen Desinfektionsphase werden praktisch alle Keime abgetötet; die Dauer der Desinfektionsphase ist als Phase der Keimabtötung verfahrensbedingt.

Anschließend folgt in Phase III eine sechsstündige Kondensationsphase, in der die relative Feuchte von mehr als 80° der feuchtheißen Desinfektionsatmosphäre reduziert wird; hierzu werden gemäß Figur 1 die Heizelemente 14 im Bodenbereich 10 abgeschaltet und ggf. noch zusätzlich Kühlelemente 15 im Bodenbereich aktiviert, so daß durch Abkühlung im Bodenbereich 10 der in der Nutzraumatmosphäre befindliche Wasserdampf wenigstens soweit kondensiert wird, daß nach Abschluß der Kondensations-Phase III nur noch eine relative Feuchte innerhalb des Nutzraumes weniger als 60 Prozent herrscht; in einer vereinfachten Ausgestaltung des Begasungsbrutschrankes kann auf die Kühlelemente 15 verzichtet werden.

An Kondensationsphase III schließt sich eine Abkühlphase IV an, wobei die Nutzraumtemperatur vom Sollwert der Desinfektion (90°C) auf den für den späteren Betrieb als Begasungsbrutschrank vorgesehenen Wert von beispielsweise 37°C abgekühlt wird. Hierzu werden zunächst die normalen Heizelemente 14 für den Nutzraum abgeschaltet, wobei lediglich die die Öffnung 7 abschließende transparente Innentür 8 (Glastür) weiterhin beheizt wird, um eine Kondensatbildung auf der Innentür zu vermeiden. Die Dauer der Abkühlphase IV liegt im Bereich von 4 bis 5 Stunden je nach Umgebungstemperatur.

An die Abkühlphase schließt sich eine mit V dargestellte Nachheizphase von beipsielsweise 3 Stunden an, bei der eine eventuell auftretende Kondensatbildung an der Innentür 8 und den Wandungen des Innenraumes zur Erreichung trockener Wand-, Decken- und Glastür-Flächen beseitig wird; die Phasen IV und V sind in ihrer Dauer gerätebedingt.

Als besonders vorteilhaft erweist es sich, daß nach dem Verfahren gemäß Figur 2 eine Dekontamination inklusive aller Einbauten und Sensoren innerhalb von Innenraum 5 erzielt wird; weiterhin erweist es sich als vorteilhaft, daß das Desinfektionsverfahren in einer Atmosphäre vorgenommen wird, deren Druck dem der umgebenden Atmosphäre weitestgehend entspricht. Ferner läuft der Desinfektionsvorgang temperatur- und zeitgesteuert automatisch ab.

## Patentansprüche

1. Verfahren zum Erzeugen einer desinfizierenden, heißen Atmosphäre im Innenraum (5) eines Begasungsbrutschrankes mittels Wärmezufuhr, wobei vor der Wärmezufuhr eine Wassermenge in den Innenraum (5) eingebracht wird, und nach Abschluss des Innenraums (5) gegenüber der Umgebungsatmosphäre in einer Aufheizphase (I) der Wassermenge Wärme zur Bildung von Wasserdampf zugeführt wird,
**dadurch gekennzeichnet,**
**dass** in der Aufheizphase (I) zusätzlich Wärme zur direkten Erhitzung von Atmosphäre und Innenraum-Oberflächen zugeführt wird und dass nach Erreichen einer Temperatur von 90°C und einer relativen Feuchte von mehr als 80 % wobei im Wesentlichen ein dem Druck der Umgebungsatmosphäre entsprechender Druck eingehalten wird, diese Atmosphäre d.h. 90°C, mehr als 80% relative Feuchte und Ungebungsdruck im Innenraum (5) für eine Desinfektionsphase (II) von wenigstens 9 h aufrechterhalten wird.

2. Verfahren nach Anspruch 1,
**dadurch gekennzeichnet,**
**dass** eine Wassermenge von wenigstens 300 ml in den Innenraum (5) eingebracht wird.

3. Verfahren nach Anspruch 2,
**dadurch gekennzeichnet,**
**dass** die Wassermenge in einen Innenraum (5) mit einem Volumen im Bereich von 15 I bis 400 I eingebracht wird.

4. Verfahren nach einem der Ansprüche 1 bis 3,
**dadurch gekennzeichnet,**
**dass** während der Aufheizphase (I) zwecks Erreichen der Desinfektionstemperatur Wärme über die gesamten Innenraum-Oberflächen zugeführt wird.

5. Verfahren nach einem der Ansprüche 1 bis 4,
**dadurch gekennzeichnet,**
**dass** während der Desinfektionsphase (II) eine Kontrolle über den gasdichten Abschluss eines Nutzraumes (6) erfolgt, wobei eine zwischenzeitliche Öffnung zu einem automatischen Neustart der Desinfektionsphase (II) führt.

6. Verfahren nach Anspruch 5,
**dadurch gekennzeichnet,**
**dass** sich an die Desinfektionsphase (II) eine Kondensationsphase (III) anschließt, wobei wenigstens in einem Bodenbereich (10) des Nutzraumes (6) eine Abkühlung erfolgt und rekondensierter Wasserdampf im Bodenbereich (10) als Wasser gesammelt wird.

7. Verfahren nach Anspruch 6,
**dadurch gekennzeichnet,**
**dass** im Innenraum (5) bei gleicher Temperatur, wie während der Desinfektionsphase (II), die relative Feuchte während der Kondensationsphase (III) um wenigstens 20 % reduziert wird.

8. Verfahren nach Anspruch 6 oder 7,
**dadurch gekennzeichnet,**
**dass** sich an die Kondensationsphase (III) eine Abkühlphase (IV) anschließt, wobei die Temperatur des Nutzraumes (6) auf einen ursprünglich eingestellten Temperatur-Sollwert abfällt.

9. Verfahren nach Anspruch 8,
**dadurch gekennzeichnet,**
**dass** in einer sich an die Abkühlphase (IV) anschließenden Nachheizphase (V) ein zuvor gebildetes Restkondensat von den Innenraum-Oberflächen weitgehend entfernt wird.

10. Begasungsbrutschrank mit einem beheizbaren Nutzraum (6), dessen Innenraum befeuchtbar ist, wobei wenigstens im Bodenbereich (10) sowie im Bereich wenigstens einer Seitenwand (12) und/oder im Deckenbereich (13) des Nutzraumes (6) jeweils wenigstens ein Temperierelement (14, 15) vorgesehen ist und die Temperierelemente (14, 15) im Bodenbereich (10) separat von den übrigen ansteuerbar sind,
**dadurch gekennzeichnet,**
**dass** der Bodenbereich (10) zur Aufnahme von Flüssigkeit ausgebildet ist und dass Druckausgleichsmittel (20, 23) vorgesehen sind, die den Druck im Inneren des Nutzraumes (6) im Wesentlichen an den Druck der umgebenden Atmosphäre angleichen.

11. Begasungsbrutschrank nach Anspruch 10,
**dadurch gekennzeichnet,**
**dass** die dem Bodenbereich (10) zugeordneten Temperierelemente (14, 15) aus Heiz- und/oder Kühlelementen bestehen.

12. Begasungsbrutschrank nach Anspruch 10 oder 11,
**dadurch gekennzeichnet,**
**dass** der Nutzraum (6) eine frontseitige Öffnung (7) aufweist, die mittels einer Innen-Tür (8) gasdicht abschließbar ist, die zur Kondensatvermeidung temperierbar ist.

13. Begasungsbrutschrank nach Anspruch 12,
**dadurch gekennzeichnet,**
**dass** die Innen-Tür (8) wenigstens zum Teil transparent ausgebildet ist.

## Claims

1. A procedure for producing a disinfecting, hot atmosphere in the inside space (5) of a gassing incubator by means of adding heat, with a quantity of water being introduced into the inside space (5) before heat is being added, and after the inside space (5) has been sealed to the surrounding atmosphere, heat being added to the quantity of water for the formation of water vapor during a heat-up phase (I),
**characterized in that**
during the heat-up phase (I), in addition, heat is added for directly heating the atmosphere and the inside space surfaces and that after a temperature of 90°C and a relative humidity of more than 80% has been reached, this atmosphere, i.e. 90°C, more than 80 % relative humidity and ambient pressure, is maintained in the inside space (5) for a disinfection phase (II) of at least 9 hours, with a pressure being maintained which essentially corresponds to the pressure of the surrounding atmosphere.

2. The procedure according to claim 1, **characterized in that** a quantity of at least 300 ml of water is introduced into the inside space (5).

3. The procedure according to claim 2, **characterized in that** the quantity of water is introduced into an inside space (5) with a volume ranging from 15 I to 400 I.

4. The procedure according to one of claims 1 to 3, **characterized in that** during the heat-up phase (I) heat is added via the entire inside space surfaces in order to reach the disinfecting temperature.

5. The process according to one of claims 1 to 4, **characterized in that** during the disinfection phase (II) the gas-tight sealing of a useful space (6) is checked, with an interim opening resulting in an automatic restarting of the disinfection phase (II).

6. The procedure according to claim 5, **characterized in that** the disinfection phase (II) is followed by a condensation phase (III), with cooling being effected at least in a floor area (10) of the useful space (6) and recondensated water vapor being collected as water in the floor area (10).

7. The procedure according to claim 6, **characterized in that** in the inside space (5) at the same temperature as during the disinfection phase (II) the relative humidity is decreased by at least 20 % during the condensation phase (III).

8. The procedure according to claims 6 or 7, **characterized in that** the condensation phase (III) is followed by a cooling phase (IV), with the temperature of the useful space (6) dropping to an originally set nominal temperature value.

9. The procedure according to claim 8, **characterized in that** during the post-heating phase (V) which follows the cooling phase (IV) a previously formed residual condensate is extensively removed from the inside space surfaces.

10. Gassing incubator with a heatable useful space (6) the inside space of which can be humidified, with at least one temperature stabilization element (14, 15) each being provided at least in the floor area (10) and in the area of at least one side wall (12) and/or in the ceiling area (13) of the useful space (6), and with the temperature stabilization elements (14, 15) in the floor area (10) being controllable separately from the others,
**characterized in that**
the floor area (10) is designed for receiving a liquid and that means for pressure equalization (20, 23) are provided which essentially adjust the pressure inside the useful space (6) to the pressure of the surrounding atmosphere.

11. Gassing incubator according to claim 10, **characterized in that** the temperature stabilization elements (14, 15) assigned to the floor area (10) consist of heating and/or cooling elements.

12. Gassing incubator according to claims 10 or 11, **characterized in that** the useful space (6) has a front-side opening (7) which can be sealed gas-tight by means of an inside door (8) which can be temperature stabilized in order to prevent condensate.

13. Gassing incubator according to claim 12, **characterized in that** the inner door (8) is designed at least partially transparently.

## Revendications

1. Procédé pour produire une atmosphère chaude désinfectante dans l'espace intérieur (5) d'une armoire d'incubation à gaz par apport de chaleur, dans lequel une quantité d'eau est introduite dans l'espace intérieur (5) avant l'apport de chaleur et, après fermeture de l'espace intérieur (5) par rapport à l'atmosphère ambiante, de la chaleur est apportée à l'eau pendant une phase de chauffage (I) pour former de la vapeur d'eau, ***caractérisé en ce que**,* pendant la phase de chauffage (I), de la chaleur supplémentaire est introduite pour chauffer directement l'atmosphère et les surfaces de l'espace intérieur et *e**n ce qu**'* après avoir atteint une température de 90°C et une humidité relative supérieure à 80 %, en maintenant une pression correspondant pour l'essentiel à la pression de l'atmosphère ambiante, cette atmosphère, à savoir 90°C, plus de 80 % d'humidité relative et pression ambiante, est maintenue dans l'espace intérieur (5) pendant une phase de désinfection (II) d'au moins 9 h.

2. Procédé selon la revendication 1, ***caractérisé en ce* qu'**une quantité d'eau d'au moins 300 ml est introduite dans l'espace intérieur (5).

3. Procédé selon la revendication 2, ***caractérisé en ce que*** la quantité d'eau est introduite dans un espace intérieur (5) ayant un volume compris entre 151 et 4001.

4. Procédé selon l'une quelconque des revendications 1 à 3, ***caractérisé en ce que**,* pendant la phase de chauffage (I), de la chaleur est introduite sur toutes les surfaces de l'espace intérieur afin d'atteindre la température de désinfection.

5. Procédé selon l'une quelconque des revendications 1 à 4, ***caractérisé en ce que**,* pendant la phase de désinfection (II), un contrôle de la fermeture hermétique aux gaz d'un espace utile (6) a lieu, une ouverture temporaire conduisant à un redémarrage automatique de la phase de désinfection (II).

6. Procédé selon la revendication 5, ***caractérisé en ce que*** la phase de désinfection (II) est suivie d'une phase de condensation (III), un refroidissement ayant lieu au moins dans une zone de fond (10) de l'espace utile (6) et la vapeur d'eau recondensée étant recueillie dans la zone de fond (10) sous forme d'eau.

7. Procédé selon la revendication 6, ***caractérisé en ce que**,* dans l'espace intérieur (5) à la même température que pendant la phase de désinfection (III), l'humidité relative pendant la phase de condensation (III) est réduite d'au moins 20 %.

8. Procédé selon la revendication 6 ou 7, ***caractérisé en ce que*** la phase de condensation (III) est suivie d'une phase de refroidissement (IV), la température de l'espace utile (6) descendant jusqu'à une valeur de consigne de température réglée à l'origine.

9. Procédé selon la revendication 8, ***caractérisé en ce que**,* dans une phase de chauffage ultérieure (V) faisant suite à la phase de refroidissement (IV), une condensation résiduelle précédemment formée est en grande partie éliminée des surfaces de l'espace intérieur.

10. Armoire d'incubation à gaz avec un espace utile (6) pouvant être chauffé, dont l'espace intérieur peut être humidifié, un élément de régulation de température (14, 15) étant prévu au moins dans la zone de fond (10) ainsi qu'au niveau d'au moins une paroi latérale (12) et/ou dans la zone de plafond (13) de l'espace utile (6) et les éléments (14, 15) de régulation de température de la zone de fond (10) pouvant être commandés séparément des autres, ***caractérisée en ce que*** la zone de fond (10) est conformée pour recevoir un liquide et ***en ce que*** des moyens d'équilibrage de pression (20, 23) sont prévus, qui équilibrent sensiblement la pression à l'intérieur de l'espace utile (6) avec la pression de l'atmosphère ambiante.

11. Armoire d'incubation à gaz selon la revendication 10; ***caractérisée en ce que*** les éléments (14, 15) de régulation de température associés à la zone de fond (10) sont composés d'éléments chauffants et/ou réfrigérants.

12. Armoire d'incubation à gaz selon la revendication 10 ou 11, ***caractérisée en ce que*** l'espace utile (6) présente une ouverture avant (7) qui peut être fermée de manière hermétique aux gaz au moyen d'une porte intérieure (8) qui peut être régulée en température afin d'éviter la formation de condensation.

13. Armoire d'incubation à gaz selon la revendication 12, ***caractérisée en ce que*** la porte intérieure (8) est réalisée au moins partiellement transparente.
